(19) Europäisches Patentamt
European Patent Office
Office européen des brevets

(11) Publication number: **0 046 377 A1**

# (12) EUROPEAN PATENT APPLICATION

(21) Application number: **81303698.5**

(22) Date of filing: **13.08.81**

(51) Int. Cl.³: **C 07 D 513/04**, A 61 K 31/425 // (C07D513/04, 277/00, 235/00)

(30) Priority: **16.08.80 GB 8026732**
**23.12.80 GB 8041296**
**31.01.81 GB 8103021**

(43) Date of publication of application: **24.02.82**
**Bulletin 82/8**

(84) Designated Contracting States: **BE CH DE FR IT LI NL**

(71) Applicant: **BEECHAM GROUP LIMITED, Beecham House Great West Road, Brentford, Middlesex (GB)**

(72) Inventor: **Dorgan, Roderick John, Lingworth Scotts Hill, Outwood Surrey (GB)**

(74) Representative: **Russell, Brian John et al, European Patent Attorney Beecham Pharmaceuticals Great Burgh Yew Tree Bottom Road, Epsom Surrey, KT18 5XQ (GB)**

(54) **Cycloalkenylcarboxamido derivatives of tetramisole, their preparation and use in medicine.**

(57) Compounds of formula (I):

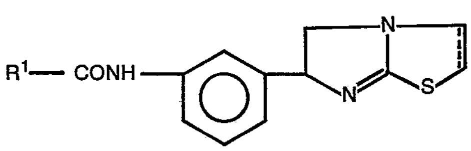

(I)

Wherein $R^1$ is $C_{5-7}$ cycloalkenyl optionally substituted with $C_{1-4}$ alkyl groups and/or a $C_5$ or $_6$ spirocycloalkyl or - alkenyl group, are novel and active against helminths. They are produced by conventional methods and formulated conventionally for use in treating humans and animals.

EP 0 046 377 A1

# Cycloalkenylcarboxamido Derivatives of Tetramisole, their preparation and use in Medicine

## ANTHELMINTICS

This invention relates to novel compounds having anthelmintic activity, to processes for their preparation and to pharmaceutical compositions containing them.

Tetramisole and levamisole are very well known anthelmintics of structure:

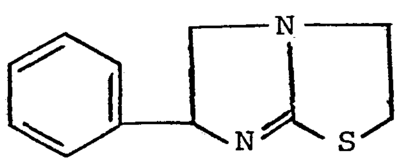

(A)

(tetramisole is the dl form, levamisole the l form).

Over the years since these anthelmintics first became known numerous publications have been made on analogues thereof. A study of these publications reveals that one class of active analogues are those of formula:

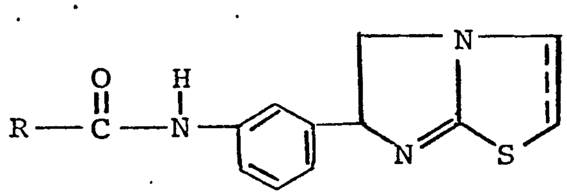

(B)

wherein the dotted line is an optionally present bond, and wherein R is, for example, alkyl (Belgian Patent No. 764755), pyridyl (U.K. Patent No. 1 516 938), isoxazolyl (Belgian Patent No. 859950), and a whole range of substituents including alkyl, aromatic and heteroaromatic groups (Belgian Patent No. 773062).

A group of novel compounds having anthelmintic activity has now been found. In particular these compounds have activity against nematodes. Surprisingly, compounds within this group are active against liver flukes such as Fasciola hepatica, and show filaricidal, especially macrofilaricidal activity.

According to the present invention, there is provided a compound of formula (I):

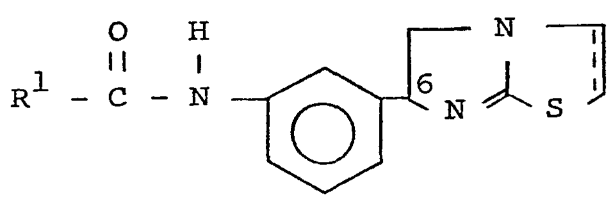

(I)

having the S-absolute configuration at the 6-position or a salt thereof,

wherein the dotted line represents an optionally present double bond;

and $R^1$ is a $C_{5-7}$ cycloalkenyl group having one or two double bonds and optionally substituted with

(i) one or more $C_{1-4}$ alkyl groups

and/or

(ii) one spirocycloalkyl or spirocycloalkenyl group formed by an optionally mono-unsaturated tetra- or pentamethylene moiety.

Compounds of formula (I) have an asymmetric carbon atom at the 6-position shown above. It is believed that anthelmintic activity isassociated with those compounds of formula (I) wherein the 6-position has S-absolute configuration. 6-S-Tetramisole is laevorotatory and, in general, the compounds of formula (I) having 6-S-configuration are also laevorotatory. However, certain compounds of formula (I) may have additional asymmetric carbon atoms and optically pure isomers of such compounds could be dextrorotatary despite having the 6-S-configuration. It is to be understood that the present invention encompasses all compounds of formula (I) having the 6-S-configuration, and such compounds may be optically pure or admixed with stereoisomers, including stereoisomers having the 6-R-absolute configuration.

Any carbon atom of the cycloalkenyl ring may bear an alkyl substituent. Saturated carbon atoms in the cycloalkenyl ring may bear one or two alkyl substituents. In addition, or alternatively, any saturated carbon atom of the cycloalkenyl ring may bear the optionally mono-unsaturated tetra- or pentamethylene moiety thus forming a spirocycloalkyl or spirocycloalkenyl group.

Suitably $R^1$ is unsubstituted or substituted with up to two alkyl groups and/or a spirocycloalkyl group.

Preferably $R^1$ is a substituted or unsubstituted cyclohexenyl or cyclopentenyl group.

Preferably any alkyl substitutents are methyl groups.

Most preferably $R^1$ is an unsubstituted cyclohexenyl group.

Suitable salts of the compounds of formula (I) include the hydrochloride, hydrobromide, hydroiodide, nitrate, sulphate, acetate, lactate, maleate, pamoate and citrate salts.

In a particular aspect, the present invention provides the first medical use of compound of formula (I) as hereinbefore defined. Alternatively, the invention provides a compound of formula (I) for use in the treatment of the human or animal body, especially for the treatment or prophylaxis of helminthiasis, particularly of humans and animals of economic importance, such as cattle, sheep and companion animals, such as dogs and cats.

According to a further aspect of the present invention there is provided a process for the preparation of a compound of formula (I) as hereinbefore defined, which process comprises reacting a compound of formula (II).

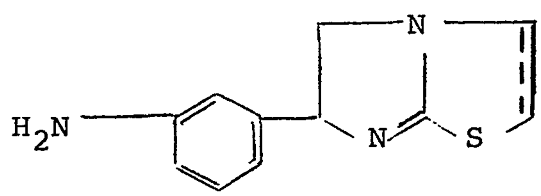

(II)

wherein the dotted line is an optionally present double bond, with an acid of formula (III)

$$R^1 - COOH \qquad (III)$$

or a reactive derivative thereof, wherein $R^1$ is as hereinbefore defined, and, when necessary, racemising the product.

"Reactive derivative" when used herein means a derivative of the acid of formula (III) which can be reacted with the amine of formula (II) to form an amido linkage between the acid group of the compound (III) and the amino group of the amine.

Often this reactive derivative will be the acid halide, such as the acid chloride, of the acid (III). In such cases, the reaction will normally be carried out in an inert solvent, preferably in the presence of an acid acceptor. The inert solvent can be any solvent inert to both reactants, such as acetone or dichloromethane. The acid acceptor is suitably an organic base such as a tertiary amine e.g. triethylamine, trimethylamine, pyridine and picoline, or an inorganic acid acceptor, such as calcium carbonate, sodium carbonate, potassium carbonate and the like. It should also be noted that it is possible to use certain acid acceptors as the solvent, for example organic bases.

Another useful reactive derivative of the acid of Formula (III) that may be used is an acid ester, such as a methyl, ethyl, propyl or butyl ester, in which case the reaction is normally carried out by heating the reactants together in an inert solvent such as ethylene glycol or dichloromethane, possibly in the presence of a suitable condensing reagent, such as a trialkylaluminium, especially trimethyl aluminium.

The reaction may also be carried out by forming an anhydride of the acid of Formula (III) in the usual manner, and reacting that with the amine - normally a conventional mixed anhydride will be used; or by reacting the acid of Formula (III) and the amine in the presence of a dehydrating-catalyst such as a carbodiimide, for example dicyclohexylcarbodiimide.

Conventional methods for amide formation according to the above process are further described in texts such as "The Chemistry of Amides" Ed J. Zabicky, Interscience.

The amines of formula (II), and the acids of formula (III), may be prepared in known manner.

It will be understood that compounds of formula (I) may be prepared as a mixture of 6-R- and 6-S- isomers by starting from dl-m-aminotetramisole, the amine of formula (II). If it is desired to obtain the 6-S- isomer, this may be achieved by using the pure l-m-aminotetramisole. Alternatively, the 6-S-isomer may be obtained from a mixture of 6-R and 6-S isomers by resolution in conventional manner, (as, of course, may the l-amine of formula (II) be prepared by resolution of the corresponding dl-form). Such resolution may be effected via an optically active salt formed from the racemic compound by reaction with an optically active acid such as d-tartaric acid, (-)-dibenzoyl-L-tartaric acid or d-10 camphor sulphonic acid, (-), Resolution reactions of this general nature are described in U.K. Patent No. 1 402 689 and U.S. Patents Nos. 3 463 786 and 3 673 205.

Furthermore, the 6-S- isomer of a compound of formula (I) may be obtained by reacting the 6-R amine of formula (I), i.e. d-m-aminotetramisole, with the acid of formula (III) or a reactive derivative, then racemising the product by standard methods. Pure 6-S isomer may then be produced by resolution of the 6-R-, 6-S- mixture and further 6-S- isomer generated by racemisation of the recovered 6-R- isomer.

According to the present invention in a yet further aspect, there is provided another process for preparing a compound of formula (I), as hereinbefore defined, which process comprises reacting the dl- or l-form of a compound of formula (IV):

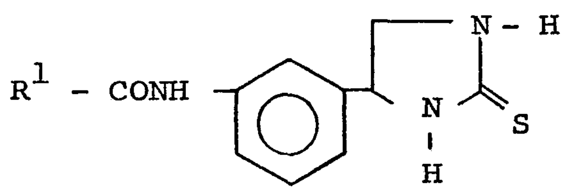

(IV)

wherein $R^1$ is as hereinbefore defined with a compound of formula (V):

$$X - CH_2 - CH_2 - Y \qquad (V)$$

wherein X and Y are the same or different and are conventional leaving groups, and, when necessary, racemising the product.

Suitably X and Y are halogen atoms or hydroxy or $C_{1-6}$ alkoxy groups.

The ring closure reaction of this process may be performed in a conventional manner using standard solvents and reaction conditions as are well known in the art.

The intermediates used in this process may be prepared by known methods.

The salts of the compounds of the formula (I) may be prepared in entirely conventional manner, for example, by reacting the compounds with the appropriate acid.

The compounds of formula (I) are useful in the treatment or prophylaxis of worm infections in humans and animals, such as cattle and sheep, cats and dogs.

Accordingly, the invention also provides a pharmaceutical or veterinary composition comprising a compound of the formula (I) or a pharmceutically or veterinarily acceptable salt thereof, and a pharmaceutically or veterinarily acceptable carrier therefor.

For oral administration the compositions may conveniently be in the form of solutions or suspensions of the active ingredient. Alternatively, the active ingredient may be mixed in with an animal feedstuff or animal feed supplement.

The compositions may also be in a form suitable for topical application with conventional carriers such as DMSO

Also the compounds of formula (I) may be administered orally to animals in sustained release formulations such as the rumen devices described in Offenlegungsschrift No. 2824288 and U.K. Patent Application No. 43555/78 (Published European Patent Application No. 0 010 987). The former devices suitably weigh about 2 to 4 g for sheep and 5 to 10 g for cattle and contain 30 to 75% by weight of a compound of formula (I). The latter devices, which suitably weigh from 5 to 10 g for sheep and 6 to 20 g for cattle would generally contain 15 to 40% by weight,

suitably 20 to 35%, for example 30%, of a compound of formula (I).

For parenteral administration the carrier will be any vehicle conveniently used for such administration, for example sterile water, or preferably a formulation in which the carrier comprises from 55 to 85% v/v propylene glycol, 0 to 5% benzylalcohol and sterile water to 100% of the carrier.

Suitably the compositions consist of sufficient material to provide a dose of from 0.1 to 100 mg of active ingredient per kg of animal body weight per dose, more suitably 1 to 50 mg/kg per dose.

The invention also provides a method of treatment or prophylaxis of helminth infections in humans or animals, which comprises the administration to the infected or potentially infected human or animal of an effective nontoxic amount of a compound of formula (I) or a pharmaceutically or veterinarily acceptable salt thereof.

In particular aspects this method includes the treatment or prophylaxis of roundworm, liver fluke and filarial infections.

It will be appreciated that it will, in some cases, be advisable to repeat the dosing of the infected or potentially infected human or animal with the compound of formula (I) or a pharmaceutically or veterinarily acceptable salt thereof according to conventional dosage regimes normally used with anthelmintics such as tetramisole and levamisole.

The following Examples illustrate the invention:

EXAMPLE 1

6/‾3-(3-cyclohexen-1-yl carboxamido)phenyl 7-2,3,5,6-tetrahydroimidazo-/‾2,1-b 7thiazole

Trimethylaluminium (10 $cm^3$ of 25% w/v solution in hexane) was added to a stirred solution of 6-(3-aminophenyl)-2,3,5,6-tetrahydroimidazo/‾2,1-b 7 thiazole (3.0 g) in dichloromethane (100 $cm^3$) at room temperature and under a nitrogen atmosphere. The mixture was stirred for 20 minutes until methane evolution ceased. Methyl 3-cyclohexen-1-yl carboxylate (1.92 g) in dichloromethane (25 $cm^3$) was added, and the mixture heated under reflux for 16 hours.

2M Hydrochloric acid was added dropwise to neutralise the reaction mixture, which was then basified with 5% ammonia solution. The resulting mixture was extracted with chloroform (3 x 200 $cm^3$), and the combined organic extracts dried ($MgSO_4$), and evaporated *in vacuo* to give a pale yellow oil (4.6 g). This was chromatographed (alumina; chloroform) to give 6/‾3-(3-cyclohexen-1-yl carboxamido)phenyl 7-2,3,5,6-tetrahydroimidazo/‾2,1-b 7thiazole (3.0 g, 67%), m.p. 129-131°C (from toluene).

EXAMPLE 2

6-/̄3-(3,3-dimethyl-1,5-cyclohexadien-1-yl carboxamido) phenyl 7̲-2,3,5,6-tetrahydroimidazo/̄2,1-b 7̲thiazole hydrochloride

Trimethylaluminium (6 ml of 25% w/v solution hexane) was added to a stirred solution of 6(3-aminophenyl)-2,3,5,6-tetrahydroimidazo/̄2,1-b 7̲ thiazole (2.2 g) in dichloromethane (50 ml) at room temperature under a nitrogen atmosphere. The mixture was stirred for 20 minutes and methyl 3,3-dimethyl-1,5-cyclohexadien-1-yl carboxylate (1.7 g) was added. The mixture was heated under reflux for 24 hours and then excess dilute hydrochloric acid added. The mixture was then basified with ammonia solution and extracted with chloroform (3 x 200 ml). The extracts were dried and evaporated, and the residue was purified by column chromatography (alumina/chloroform).

Treatment of the product with hydrogen chloride in isopropanol gave 6-/̄3-(3,3-dimethyl-1,5-cyclohexadien-1-ylcarboxamide )phenyl 7̲-2,3,5,6-tetrahydroimidazo /̄2,1-b 7̲thiazole hydrochloride (1.8 g), m.p. 169-71°C.

EXAMPLE 3

6-/‾3-(1,4-cyclohexadien-1-yl carboxamido)phenyl_7-2,3,5,6-tetrahydroimidazo/‾2,1-b_7thiazole

By the method of Examples 1 and 2, but using 6-(3-aminophenyl)-2,3,5,6-tetrahydroimidazo/‾2,1-b_7 thiazole and methyl 1,4-cyclohexadien-1-yl carboxylate, 6-/‾3-(1,4-cyclohexadien-1-yl carboxamide)phenyl_7-2,3,5,6-tetrahydroimidazo/‾2,1-b_7thiazole was prepared, m.p. 118-120°C (from ethyl acetate).

EXAMPLE 4

6-[3-(2-cyclopenten-1-yl carboxamido)phenyl]-2,3,5,6-tetrahydroimidazo[2,1-b]thiazole

By the method of Examples 1 and 2, but using 6-(3-aminophenyl-2,3,5,6-tetrahydroimidazo[2,1-b]thiazole and methyl 2-cyclopenten-1-yl carboxylate, 6-[3-(2-cyclopenten-1-yl carboxamido)phenyl]-2,3,5,6-tetrahydroimidazo[2,1-b] thiazole was prepared, as colourless crystals, m.p. 115-6°C(from ethyl acetate).

0046377

Examples 5 - 12

By the method of Examples 1 and 2, but using the appropriate starting materials, the following compounds were prepared.

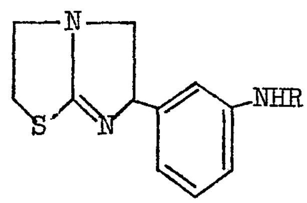

| Example No. | R | m.p. (°C) |
| --- | --- | --- |
| 5 | 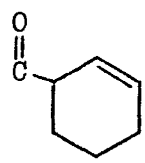 | 123-5 |
| 6 | 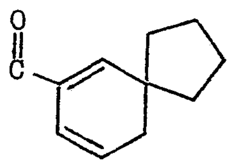 | 110-112 |
| 7 | 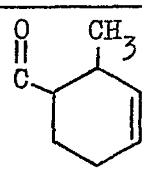 | 137-9 |
| 8 | 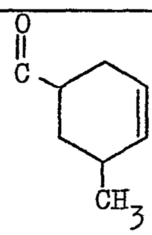 | 141-3 |

| Example No. | R | mp (°C) . |
|---|---|---|
| 9 | 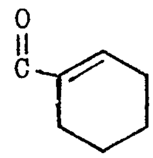 | 91-3 |
| 10 | 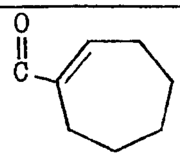 | 118-119.5 |
| 11 | 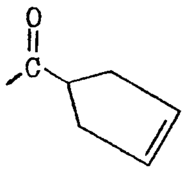 | 135-6 |
| 12 | 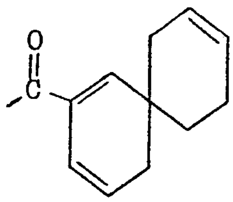 | 92-5 |

ANALYTICAL DATA

Compound
of Ex No

1 Found:- C: 66.5; H: 6.7; N: 12.7; S: 9.8%
$C_{18}H_{21}N_3OS$
requires:- C: 66.0; H: 6.5; N: 12.8; S: 9.8%

2 Found:- C: 59.77; H: 5.44; N: 10.29; S: 7.92%
$C_{20}H_{23}N_3SO$:1.2 HCl
requires:- C: 60.40; H: 6.09; N: 10.57; S: 8.05%

4 Found:- C: 64.8; H: 5.8; N: 13.1. S: 10.4%
$C_{17}H_{19}N_3SO$
requires:- C: 65.1; H: 6.1; N: 13.4; S: 10.2%

5 Found:- C: 66.1; H: 6.1%
$C_{18}H_{21}N_3SO$
requires:- C: 66.1, H: 6.4%

6 Found:- C: 68.9; H: 5.9; N: 10.6%
$C_{22}H_{25}N_3SO$
requires:- C: 69.6; H: 6.6; N: 11.0%

7 Found:- C: 66.6; H: 6.8; N: 12.6%
$C_{19}H_{23}N_3OS$
requires:- C: 66.8; H: 6.8; N: 12.3%

8 Found:- C:66.6; H: 6.7; N: 12.4%
$C_{19}H_{23}N_3SO$
requires:- C: 66.8; H: 6.8; N: 12.3%

| Compound of Ex No | | |
|---|---|---|
| 10 | Found:- | C: 66.6; H: 6.9; N: 12.4; S: 9.4% |
| | $C_{19}H_{23}N_3SO$ requires | C: 66.8; H: 6.8; N: 12.3; S: 9.4% |
| 11 | Found:- | C: 64.5, H: 6.1; N: 13.2% |
| | $C_{17}H_{19}N_3OS$ requires:- | C: 65.1; H: 6.1; N: 13.4% |

EXAMPLE 13

Preparation of 6-[3-(3-cyclohexen-1-ylcarboxamido)phenyl]-2,3,5,6-tetrahydroimidazo[2,1-b]thiazole

Cyclohex-3-ene carboxylic acid (18.9 g, 0.15 mole) in dichloromethane (400cm$^3$) was cooled to 0$^o$C and stirred during the rapid addition of triethylamine (15.2 g, 0.15 mole), giving a straw coloured solution. Methyl chloroformate (14.2 g, 0.15 mole) was added over a period of 15 minutes, forming a white precipitate. The reaction mixture was allowed to warm to room temperature, and stirred for a further 30 minutes.

The solvent was removed in vacuo giving a white solid, to which was added a solution of the amine in aqueous methanol at pH 6. This solution was prepared by dissolving 6-(m-aminophenyl)-2,3,5,6-tetrahydroimidazo[2,1-b]thiazole hydrochloride (25.5 g, 0.1 mole) in water (100 cm$^3$) and methanol (200 cm$^3$), adjusting the pH of the solution with 2M hydrochloric acid solution and making up the volume to 400 cm$^3$ with water.

The resulting mixture was stirred vigorously at room temperature for 2 hours. During this time gas was evolved and the temperature rose slightly. The pH of the reaction mixture was maintained at ~6 by the periodic addition of 2M sodium hydroxide solution.

The solution was then basified with 0.88 ammonia solution giving a white precipitate. The mixture was extracted with dichloromethane (3 x 200 cm$^3$) and the combined extracts washed with dilute ammonia solution (200 cm$^3$). The organic layer was dried (magnesium sulphate) and evaporated in vacuo to give a white solid (35.5 g).

Crystallisation from ethyl acetate gave 6-[3-(3-cyclohexen-1-ylcarboxamido)phenyl]-2,3,5,6-tetrahydroimidazo[2,1-b]thiazole, m.p. 129-30 $^o$C.

EXAMPLE 14

Resolution of 6-[3-(3-cyclohexen-1-ylcarboxamido)phenyl]-2,3,5,6-tetrahydroimidazo[2,1-b]thiazole

Racemic 6-[3-(3-cyclohexen-1-ylcarboxamido)phenyl]-2,3,5,6-tetrahydroimidazo[2,1-b]thiazole (1.65 g) was dissolved in methanol (8 $cm^3$) and added to (-)2,3-dibenzoyl-L-tartaric acid (1.88 g) in methanol (12 $cm^3$). After 5 minutes a white precipitate began to form and the mixture was cooled to $0^oC$ for 2 hours. The precipitate was collected by filtration and washed with cold methanol (10 $cm^3$). After drying, the yield of salt was 1.7 g, m.p. 172-173 $^oC$.

This was recrystallised from a mixture of dimethylformamide (8 $cm^3$) and water (4 $cm^3$), and after filtration and drying gave 1.2 g of salt. It exhibited $[\alpha]_D^{21}$ of $-65^o \pm 5^o$ (c = 1, DMSO).

| | |
|---|---|
| Found:- | C: 62.93; H: 4.87; N: 6.10; S: 4.43% |
| $C_{36}H_{35}O_9N_3S$ requires:- | C: 63.07; H: 5.11; N: 6.13; S: 4.67% |

m.p. $176.5-177.5^oC$.

The above salt was basified with aqueous sodium hydroxide and extracted with dichloromethane (3 x 50 ml) to yield the free, laevorotary 6-[3-(3-cyclohexen-1-ylcarboxamido)phenyl]-2,3,5,6-tetrahydroimidazo[2,1-b]thiazole (0.6 g) $[\alpha]_D^{21}$ $-70^o \pm 5^o$ (c = 2, $CH_3OH$).

Similarly the dextrorotary amide $[\alpha]_D^{21}$ $+ 66^o \pm 5^o$ (c = 2, $CH_3OH$) may be obtained by using (+) 2,3-dibenzoyl-D-tartaric acid in the above procedure.

EXAMPLE 15

## 6-[3-(3-Cylohexen-1-ylcarboxamido)phenyl]-5,6-dihydroimidazo [2,1-b]-thiazole

6-[3-(3-Cyclohexen-1-ylcarboxamido)phenyl]-5,6-dihydroimidazo [2,1-b]-thiazole, m.p. 121-122°C, was prepared according to the method of Example 1 from 6-(3-aminophenyl)-5,6-dihydroimidazo[2,1-b]thiazole and methyl 3-cyclohexen-1-ylcarboxylate.

| | |
|---|---|
| Found:- | C: 66.0; H: 6.0; N: 12.9% |
| $C_{18}H_{19}N_3OS$ requires:- | C: 66.4; H: 5.9; N: 12.9% |

Pharmacological Data Section

The compounds of Examples 1 to 15 were each administered orally to mice infected with *Nematospiroides dubius* at various dosages. Activity was calculated as % reduction in the numbers of worms in treated as opposed to control (untreated, infected) animals. No obvious signs of toxicity were observed.

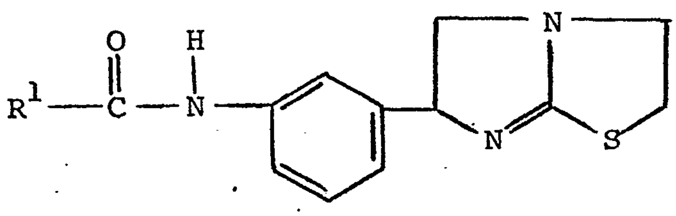

(I)

| Compound of Example | $R^1$ | Dose mg/kg | Activity % |
|---|---|---|---|
| 1 | 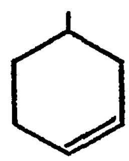 Free base | 50<br>5 | 99<br>55 |
| 2 | 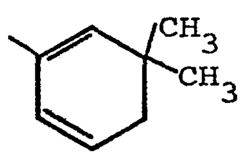 1.2HCl | 50<br>5 | 99<br>57 |
| 3 | 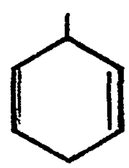 Free base | 50<br>5 | 100<br>58 |
| 4 | 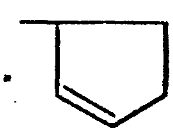 Free base | 50<br>5 | 100<br>100 |

Pharmacological Data Section

| Compound of Example | R' | Dose mg/kg | Activity % |
|---|---|---|---|
| 5 | 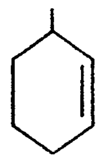 | 50<br>5 | 100<br>65 |
| 6 | 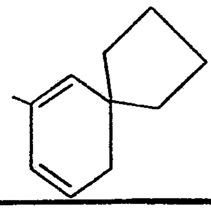 | 50<br>5 | 100<br>45 |
| 7 | 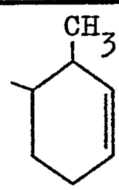 | 50<br>5 | 100<br>55 |
| 8 | 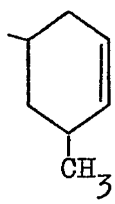 | 50<br>5 | 99<br>59 |
| 9 | 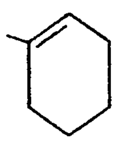 | 50<br>5 | 99<br>76 |
| 10 | 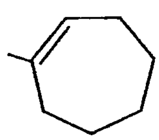 | 50<br>5 | 100<br>90 |

| Compound of Example | R' | Dose mg/kg | Activity % |
|---|---|---|---|
| 11 | 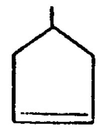 | 50<br>5 | 100<br>84 |
| 12 | 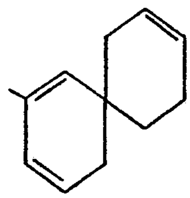 | 50<br>5 | 94<br>19 |
| 14a | l isomer 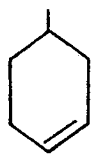 | 10<br>2.5 | 100<br>100 |
| 14b | d isomer 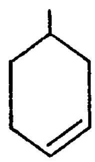 | 10 | 0 |

Dihydroimidazothiazoles

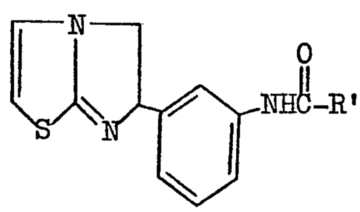

| Compound of Example | R' | Dose mg/kg | Activity % |
|---|---|---|---|
| 15 | 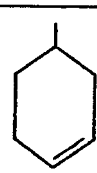 | 50<br>5 | 100<br>71 |

Test to Demonstrate Fluke Activity

A sheep was artificially infected with 100 metacercariae and 4 - 5 months later was treated, orally, with 6-[3-(3-cyclohexen-1-ylcarboxamido)-phenyl]-2,3,5,6-tetrahydroimidazo[2,1-b]thiazole at 30 mg/kg. Fluke egg counts fell to zero 3 - 4 days after dosing, and post-mortem fluke worm counts revealed an activity > 95%.

Activity against Sheep Roundworms.

a) By Oral Route

A single oral dose of 15 mg/kg of the compound of Example 1, as an aqueous solution of its hydrochloride salt, to a sheep with a mixed roundworm infection removed all roundworms present.

b) By Subcutaneous Route

A single subcutaneous injection of 5 mg/kg of the laevo amide of Example 12a, as a solution of its hydrochloride (in a mixture of propylene glycol, 70%; benzyl alcohol, 4%; and made up to 100% with water), to a sheep with a mixed roundworm infection, removed >99.9% of all worms present and was 100% active against lungworm.

Activity against Brugia pahangi in the Cat (Macrofilaricidal activity)

Five daily doses of the laevo amide of Example 12a administered at 5 mg/kg s.c. to a cat infected with adult Brugia pahangi was 100% effective against this parasite.

Activity against Dog Parasites

A single oral dose of 7.5 mg/kg of the laevo amide of Example 12a to a dog was 100% effective against ascarids, hookworm and whipworm.

CLAIMS

1. A compound of formula (I):

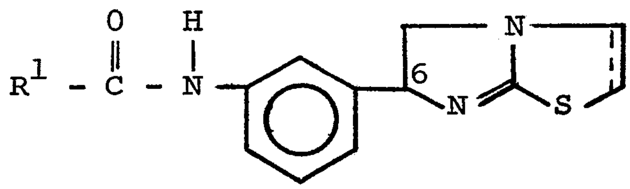

(I)

having the S-absolute configuration at the 6-position, or a salt thereof,

wherein

the dotted line represents an optionally present double bond;

and $R^1$ is $C_{5-7}$ cycloalkenyl having one or two double bonds and optionally substituted with

(a) one or more $C_{1-4}$ alkyl groups and/or

(b) one spirocycloalkyl or spirocycloalkenyl group formed by an optionally mono-unsaturated tetra- or penta- methylene moiety.

2. A compound as claimed in claim 1 wherein $R^1$ is a substituted or unsubsituted cyclohexenyl or cyclopentenyl group.

3. 6-[3-(3,3-dimethyl-1,5-cyclohexadien-1-ylcarboxamido) phenyl]-2,3,5,6-tetrahydroimidazo[2,1-b]thiazole;

6-[3-(1,4-cyclohexadien-1-ylcarboxamido)phenyl]-2,3,5,6-tetrahydroimidazo[2,1-b]thiazole;

6-[3-(2-cyclopenten-1-ylcarboxamido)phenyl]-2,3,5,6-tetrahydroimidazo[2,1-b]thiazole;

6-[3-(2-cyclohexen-1-ylcarboxamido)phenyl]-2,3,5,6-tetrahydroimidazo[2,1-b]thiazole;

6-[3-(3-spirocyclopentyl-1,5-cyclohexadien-1-ylcarboxamido)phenyl]-
2,3,5,6-tetrahydroimidazo[2,1-b]thiazole;
6-[3-(3-cyclohexen-1-yl carboxamido)phenyl]-2,3,5,6-tetrahydroimidazo-[2,1-b]thiazole
6-[3-(2-methyl-3-cyclohexen -1-ylcarboxamido)phenyl]-
2,3,5,6-tetrahydroimidazo[2,1-b]thiazole;
6-[3-(5-methyl-3-cyclohexen-1-ylcarboxamido)phenyl]-
2,3,5,6-tetrahydroimidazo[2,1-b]thiazole;
6-[3-(1-cyclohexen-1-ylcarboxamido)phenyl]-
2,3,5,6-tetrahydroimidazo[2,1-b]thiazole;
6-[3-(1-cyclohepten-1-ylcarboxamido)phenyl]-
2,3,5,6-tetrahydroimidazo[2,1-b]thiazole;
6-[3-(3-cyclopenten-1-ylcarboxamido)phenyl]-
2,3,5,6-tetrahydroimidazo[2,1-b]thiazole;
6-[3-(3-[spiro-3-cyclohexen-1-yl]-1,5-cyclohexadien-1-ylcarboxamido)phenyl]-
2,3,5,6-tetrahydroimidazo[2,1-b]thiazole;
and 6-[3-(3-cyclohexen-1-ylcarboxamido)phenyl]-
5,6-dihydroimidazo[2,1-b]thiazole.

4. A compound as claimed in any one of claims 1 to 3 and substantially free from the corresponding compound having R-absolute configuration at the 6-position.

5. A compound as claimed in any one of claims 1 to 4 for use in treating the human or animal body.

6. A process for producing a compound of formula (I) as defined in claim 1, which process comprises

(a) reacting a compound of formula (II):

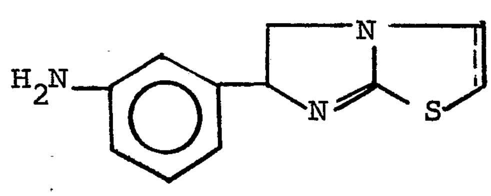

(II)

 

wherein the dotted line is an optionally present double bond, with an acid of formula (III):

$$R^1 - COOH \qquad (III)$$

or a reactive derivative thereof,

Wherein $R^1$ is as defined in claim 1;

or (b) reacting a compound of formula (IV):

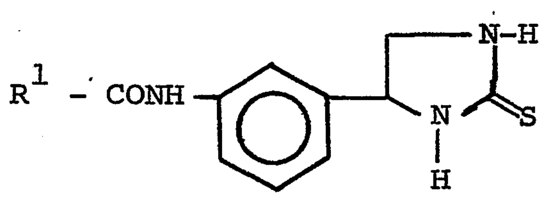

(IV)

wherein $R^1$ is as defined in claim 1;

with a compound of formula (V):

$$X - CH_2 - CH_2 - Y \qquad (V)$$

wherein X and Y are the same or different and are conventional leaving groups;
and, when necessary, racemising the compound so formed.

7. A veterinary composition comprising a compound of formula (I) as defined in claim 1 in association with a veterinary acceptable carrier therefor.

8. A composition as claimed in claim 7 wherein the carrier is feedstuff or feed supplement provided for animals.

9. A composition as claimed in claim 7 wherein the carrier is a sustained release device.

10. A process for producing a composition as claimed in claim 7 which process comprises admixing the compound of formula (I) and the carrier therefor.

European Patent Office

# EUROPEAN SEARCH REPORT

0046377

Application number
EP 81 30 3698

## DOCUMENTS CONSIDERED TO BE RELEVANT

| Category | Citation of document with indication, where appropriate, of relevant passages | Relevant to claim |
|---|---|---|
| D | BE - A - 773 062 (AMERICAN GYANAMID COMP.)<br>* Whole document *<br>---- | 1,7 |

**CLASSIFICATION OF THE APPLICATION (Int. Cl.3)**

C 07 D 513/04
A 61 K 31/425//
(C 07 D 513/04,
277/00,
235/00)

**TECHNICAL FIELDS SEARCHED (Int. Cl.3)**

C 07 D 513/04
A 61 K 31/425

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 21-09-1981 | ALFARO |

EPO Form 1503.1 06.78